# EUROPEAN PATENT APPLICATION

(11) **EP 4 357 337 A1**
(43) Date of publication of application: **24.04.2024**
(21) Application number: 22830365.7
(22) Date of filing: 23.06.2022
(51) Int. Cl.: C07D 295/24, D01F 13/02, C02F 9/00

(54) **METHOD AND SYSTEM FOR PURIFYING NMMO AND OBTAINED NMMO HYDRATE CRYSTAL**

(30) Priority: 02.07.2021 CN 202110748233; 12.01.2022 CN 202210034607
(71) Applicant: B-FCTL Co., Ltd., Cangzhou, Hebei 061100 (CN)
(72) Inventor: LU, Wanli, Hebei 061100 (CN); MA, Jie, Shaanxi 710018 (CN)
(74) Representative: Müller Schupfner & Partner Patent- und Rechtsanwaltspartnerschaft mbB
(86) International application number: PCT/CN2022/100871
(87) International publication number: WO 2023/274037

(57) **Abstract**

Disclosed in the present invention are a purification method and system of NMMO and a NMMO hydrate crystal obtained thereof. The purification method is suitable for purifying NMMO in a coagulating bath of a cellulose product, and by means of the method, almost all impurities including carbohydrate impurities can be removed from the NMMO solution of the coagulating bath of the cellulose product and a high-purity NMMO hydrate crystal is obtained. The purification method comprises the following steps: performing a cooling crystallization to the coagulating bath of the cellulose product between -20°C and 78°C to obtain NMMO hydrate crystals. According to the method for purifying and recovering NMMO in the coagulating bath of the cellulose product in the invention, ion-exchange resins are not needed, and acid and alkali are not needed for resin regeneration. High-salt wastewater is not generated, and almost no discharge of waste gas, waste water, and solid waste. Environmental protection problems caused by a large amount of high-salt and high-COD waste water generated by ion-exchange resin regeneration and spent ion-exchange resins are solved. The preparation process for the cellulose product truly becomes a low-cost and green production process, and the method can be applied to a wider field of cellulose product production.

## Description

### FIELD

The invention relates to a purification method of N-methylmorpholine N-oxide (NMMO), a purification system of N-methylmorpholine N-oxide (NMMO) and a N-methylmorpholine N-oxide (NMMO) obtained by using the method and the system.

### BACKGROUND

Cellulose is a renewable resource widely exists in nature. Dissolving cellulose pulp directly in a NMMO aqueous solution with a mass concentration above 85%, a renewable cellulose fiber known as lyocell fiber can be obtained by spinning directly without chemical reaction. The lyocell fiber has excellent properties with simple production process, and the NMMO solvent used is non-tox. The production of lyocell fibers is closed-loop production cycle, and the NMMO solvent can be recovered for recycling. The product waste is biodegradable and does not pollute the environment. Therefore, the lyocell fibers are known as green and environmentally friendly fibers with good development prospect in the 21^{st} century. The lyocell fiber is also one of the most successful applications of preparing cellulose products by using cellulose as raw material nowadays.

The production process of the lyocell fiber is divided into two parts. The first part of the production process is cellulose dissolution and spinning in NMMO and the second part is purification and recovery of the NMMO in the coagulation bath solution after lyocell spinning. Firstly, dissolving cellulose pulp and other auxiliaries such as propyl gallate (PG) in a NMMO with a water content of 13.3% between 90 ~ 110°C to form a homogeneous cellulose fiber NMMO solution (known as `spinning solution' in the production of lyocell fiber). Then the spinning is performed by the method of dry-jet wet spinning. Filaments from the spinneret first pass through an air gap(dry-jet) and then enter the coagulation bath solution. The coagulation bath solution is a NMMO aqueous solution with low concentration and the concentration of NMMO is generally between 5 ~ 25%. Through the dilution of the coagulation bath solution, the lyocell cellulose fibers are solidified and formed. The lyocell coagulation bath solution containing low concentration of NMMO undergoes purification processes to achieve purification (the purification is generally performed by using the method of ion-exchange resins now) and is then concentrated to a required concentration by conventional heating evaporation under reduced pressure for recycling.

In addition to lyocell fiber, prior arts also relate to other preparations of cellulose products from NMMO solution, such as using the method of extrusion and biaxial elongation (blowing) to prepare cellulose tubular films (HAO Yan-ping, Properties and structure of biaxial cellulose films from NMMO solution, Master dissertation, 2007; US2003/0183976A1 ; US2002/0022100A1; US2004/0146668A1; US5597587), using the method of extrusion and elongation to prepare spiral cellulose flat membranes (CN1224435A, US6833187B2), using the method of casting and scrapping to prepare cellulose flat membranes, which is used for packaging materials and separation membranes (Yaopeng Zhang, Preparation and formation mechanism of cellulose membranes prepared from NMMO solution, Doctoral dissertation, 2002), adding bacteriostatic agent such as chitosan, thymol, gluconic acid, etc. in cellulose NMMO solution (known as `casting solution' in the membrane production process) to prepare cellulose membranes with antibacterial function (Lingling Yang, The preparation and research in controlled release ability of antimicrobial membrane based natural cellulose, Master dissertation, 2013; Hengsen Yuan, Research on preparation and properties of food packaging cellulose film, Master dissertation, 2012), preparation of cellulose sponge (Xiangiuan Jia, Preparation of High-adsorbed regenerated cellulose materials using NMMO/H2O, Master dissertation, 2016; Xiaohui Liu, Preparation of cellulose sponge by using N-methylmorpholine N-oxide (NMMO) as solvent, Master dissertation, 2013), preparation of cellulose products such as cellulose non-woven fabrics (US20170107644, US8420004), cellulose film (US7938993), cellulose material for cigarette filter (US10,306,919) and etc. Although the above cellulose products can be prepared by using NMMO as solvent, none of the above prior arts mentioned how to purify and recover the NMMO solvent in the coagulation baths.

Similar to the preparation of lyocell fiber, the above cellulose products are also prepared by dissolving cellulose in the NMMO. Taking the preparation of cellulose membranes as an example, the first part is also dissolving cellulose pulp and other necessary auxiliaries (such as PG) in a NMMO solution between 90 ~ 110°C to form a brown, homogeneous and transparent cellulose solution (known as `casting solution' in the cellulose membrane production), and then using different membrane forming method, for example the method of casting and scrapping, the method of extrusion and elongation, the method of extrusion and biaxial elongation (blowing) etc., to form the cellulose solution into membranes according to different production requirements; the second part is that the membrane-forming cellulose solution passes through an air gap and then enter the coagulation bath, and the cellulose coagulates and precipitates in the coagulation bath to form the cellulose membranes, which becomes the final cellulose membrane products after processes of washing, postprocessing (glycerin plasticizer treatment), drying etc. The NMMO solvent in the coagulation bath needs to be purified and recovered to remove various impurities contained therein and then the "pure" NMMO solution is concentrated for recycling. Due to different requirements in membrane production process, the concentration of NMMO in the coagulation bath varies significantly. Further, the types and contents of impurities contained therein also varies greatly. For instance, when the packaging films with good transparency and good mechanical properties are prepared, the lower the concentration of NMMO in the coagulation bath, the better the packaging films is. Sometimes, the concentration of NMMO is even lower than 5%. Considering economic and environmental aspects, the NMMO in the coagulation bath needs to be purified and then concentrated (such as heating concentration under reduced pressure) to a required concentration for recycling.

The preparation of other cellulose products, for example cellulose sponges, cellulose non-woven fabrics, cellulose cigarette filters, cellulose films, cellulose containers, etc., are similar to the lyocell fibers and cellulose membranes, which means they all needs to dissolve cellulose pulp and other auxiliaries (such as PG) in a NMMO aqueous solution with a concentration of 85 ~ 88% between 90 ~ 110°C to form a cellulose solution first. After the products are formed and enter an aqueous solution of NMMO coagulation bath with a low concentration, cellulose coagulates and precipitates in the coagulation bath to form corresponding cellulose products, while the NMMO in the coagulation bath also needs to be purified and recovered for recycling.

Since the method of preparing cellulose product from NMMO solution has technical difficulties in purifying and recovering NMMO in the coagulation bath, fewer other cellulose products are industrially prepared by using NMMO as a solvent except the successful industrialization of lyocell fibers. Cellulose products currently in the market are generally prepared by the method of viscose process and cuprammonium process. For example, cellophane is mainly prepared by the method of viscose process while dialysis membrane and separating membrane are prepared by the method of cuprammonium process. Both of the above two processes have problems such as complex processes, multiple production steps, long production cycle, high production cost, serious pollution emissions, etc. Furthermore, the cellulose membranes obtained thereof contain toxic and harmful substances.

A great deal of research has been conducted in academia and industry worldwide on the production of lyocell fibers. It is discovered that during the dissolution of cellulose pulp in the NMMO under a relatively high temperature of 90 ~ 110°C, NMMO molecules and cellulose molecules will have complicated chemical decomposition reactions (such as radical reaction) in the heating state and produce multiple impurities. To recycle the NMMO, various impurities in the NMMO solution of the coagulation bath need to be removed. It is reported that the NMMO may decompose into impurities, for example the N-methylmorpholine, morpholine, formaldehyde, chemical compound with large π- electron-conjugated structure such as phenols and quinoline, etc. between 90 ~ 110°C. Highly reactive radicals may also be produced during the decomposition, which leads to oxidation of the cellulose and breakdown and decomposition of cellulose chain. Metal ions, such as copper ions, iron ions etc., in the cellulose solution will catalyze and accelerate the decomposition of NMMO and the breakdown of the cellulose and deepen the color of the cellulose solution. Cellulose breakdown products include oligosaccharide with more than two saccharides and monosaccharide, and carbohydrates may decompose into a dozen of impurities such as sugar ketones, sugar acid, furans, furfurals, phenols, etc. in the meantime. There may be further reactions between these decomposition products and it has been reported that up to 20 organic impurities containing chromophores may be produced (Rosenua T., Potthast A., Milacher W., Adorjan I., Hofinger A. and Kosma P., Discoloration of cellulose solutions in N-methylmorpholine-N-oxide (Lyocell). Part 2: Isolation and identification of chromophores. Cellulose 2005,12:197-208) (ZHENG Yu-cheng, Cause analysis of color change in lyocell fiber production [J], Synthetic Fiber, 2018, 47 (8): 17-23). If the above impurities cannot be removed during the recycling of the NMMO in the coagulation bath, the continuous accumulation of the impurities over time will have a negative impact on lyocell fibers; To prevent NMMO decomposition and cellulose breakdown, multiple compound stabilizers including antioxidant of propyl gallate (PG), hydroxylamine etc., need to be added during the dissolution of cellulose by NMMO. These stabilizers, as well as the oxidized products of propyl gallate (PG) generated after scavenging the radicals, and condensation products from the reaction of PG and formaldehyde all have darker colors. Excessive residues of these chromophores in the solution will deepen the color of the final cellulose products. In addition, to remove residual suspended substance in the NMMO of the coagulation bath and reduce solution turbidity, flocculant of polyacrylamide (PAM) is usually added in the lyocell fiber production to perform flocculation and sedimentation and the suspended substance is then removed by microfiltration. However, there is still a certain amount hemicellulose, oligosaccharides, etc. have not been removed, while there is still a small amount of PAM residues in the NMMO solution of the coagulation bath. Therefore, the impurities in the coagulation bath are complex, and the above impurities dissolved in the NMMO solvents of the coagulation bath must be purified and removed when the NMMO solvents are recovered for recycling.

Like the preparation of lyocell fibers, the first step of preparations for other cellulose products, such as the preparation of cellulose membranes, is also dissolving cellulose pulps in a NMMO aqueous solution with a concentration of 85 ~ 87 % at a temperature around 100 °C to form a uniformly dissolved cellulose solution. During the dissolution at high temperatures, various impurities will be generated, and these impurities will also dissolve in the NMMO aqueous solution of the coagulation bath. Besides, to adjust the rheological property of the casting solution and the porosity of the membranes after the membranes are formed, extra auxiliaries such as bacteriostatic agent, pore-forming agent, etc., needs to be added sometimes. If these added auxiliaries cannot be removed in time during the purification of the coagulation bath, auxiliaries will be transferred to the following casting solution and accumulated. Excessive concentration of these auxiliaries may leave defects on the surface of the prepared cellulose membranes, which will not only affect mechanical properties of the formed membranes, but also has great effect on the selective separation ability of the membranes. Therefore, the various impurities must be completely removed from the NMMO coagulation bath of the cellulose membrane products by necessary purification methods before the NMMO can be recycled.

Preparation processes for other cellulose products are similar to the production of lyocell fibers and cellulose membranes. The first part is also the preparation of the cellulose NMMO solution, and the second part is that cellulose solution is molded into the shape of the desired products and then, enter the coagulation bath with low concentration of the NMMO aqueous solution and in the coagulation bath, the cellulose is precipitated and the cellulose products are formed. Required auxiliaries may need to be added when preparing different cellulose products. For instance, a large amount of pore-forming agent, such as solid anhydrous sodium sulfate, needs to be added in the preparation of cellulose sponge products and a large amount of pore-forming agent, such as calcium chloride, etc., also needs to be added in the preparation of cellulose shower loofah. Hence, NMMO solution of the coagulation bath of these cellulose products may contain a greater variety and higher concentration of impurities than the lyocell fiber coagulation bath. These impurities must be removed by necessary purification methods to realize the recycling of the NMMO solvents.

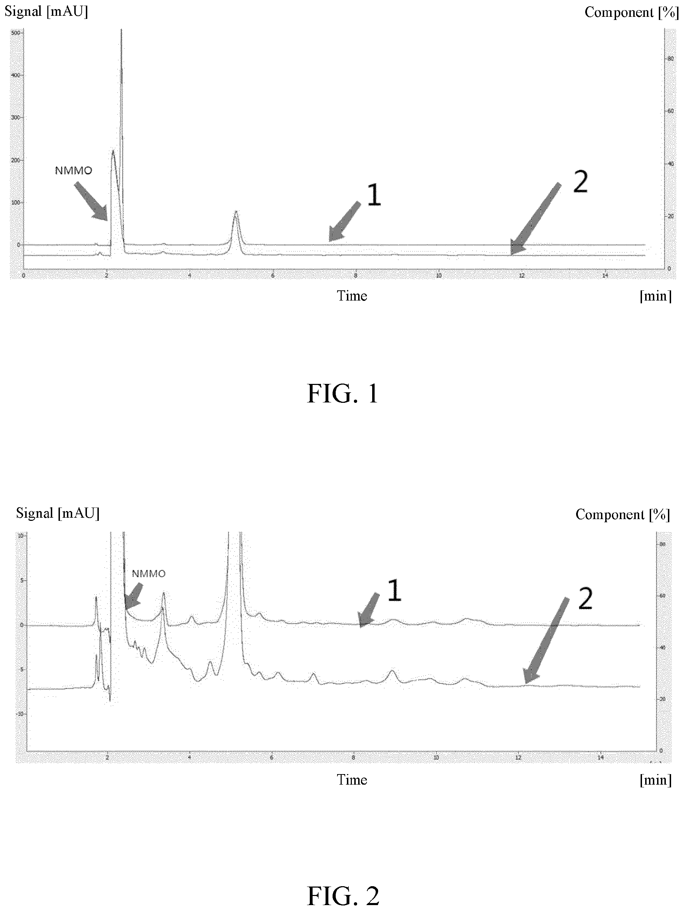

Currently, the purification and recovery processes of the NMMO in the coagulation bath in lyocell fiber preparation process mainly use the combination of anion-exchange resin and cation-exchange resin to perform purification (HAN Zeng-qiang, etc., Research and Development of the Recycling Process of NMMO for Producing Lyocell Fiber [J], Shandong Textile Science & Technology, 2007, (3):7-9) (Yue Wentao, etc., The Purification and Regeneration of the Aqueous Solutions of NMMO using Ion Exchange Resin [J], Synthetic Fiber, 2002, 31(2):28-30), but the purification and recovery processes by using ion-exchange resin have many problems: (1) the recovered and purified NMMO still contains many impurities and the purity is relatively low. The NMMO of the coagulation bath contains a number of impurities which cannot form ions in acid or base environment, such as propyl gallate (PG) and the products of its reaction, carbohydrates and decomposition products, and some of thermal decomposition products of the NMMO, etc., therefore these impurities cannot be completely removed by ion exchange resin. The applicant of the invention has performed liquid chromatography analysis (refer to Fig. 1 and 2) and total sugar analysis to the samples of lyocell coagulation bath solution before and after the purification using ion-exchange resin obtained from a Chinese lyocell manufacturer and it has been discovered that the coagulation baths contain multiple impurities including carbohydrate impurities which cannot be removed by ion-exchange resin. The purity of the NMMO of the coagulation bath after purification by ion-exchange resin alone is not high accordingly. In addition, since the ion-exchange resin cannot remove carbohydrates from the coagulation bath, most of the lyocell manufacturers have to use coniferous forest pulps with a very low content of hemicellulose (β-cellulose) as raw materials for lyocell fiber while cheap and easily obtained broad-leaved forest pulps are not able to be used since the broad-leaved forest pulps contain higher hemicellulose content. The hemicellulose breaks and decomposes more easily in the lyocell condition compared to α-cellulose and produces more carbohydrate impurities. If these carbohydrate impurities cannot be removed in time, carbohydrate impurities may be continuously accumulated in the purified and recovered NMMO and finally affect the stable production of lyocell fibers; (2) There exist the pollution problems from the significant amount of wastewater with high concentration of salt and COD content in lyocell production. Exhausted ion-exchange resins need to be regenerated, which means cation exchange resins require a 4-6% hydrochloric acid aqueous solution that is 4-6 times the volume of the resins for regeneration, while anion exchange resins require a 4-6% NaOH aqueous solution that is 4-6 times the volume of the resins for regeneration. The waste liquid from resin regeneration contains not only high content of salt but also a significant amount of organic impurities, which leads to high treatment costs. Generally, producing 1 ton of lyocell fibers will have to generate 10-15 tons of the resin regeneration waste liquid. (3) Relatively high production costs. Ion-exchange resins suitable for purification of NMMO in the lyocell fiber coagulation bath are expensive and have lifespan of only 1-2 years. The cost of lyocell fibers apportioned per ton is 500 CNY. According to the national hazardous waste classification list, spent ion-exchange resins are classified as hazardous waste and must be disposed of at a high cost by qualified environmental protection companies.

The production of cellulose products, especially the cellulose membranes, requires higher NMMO purity than the production of lyocell fibers. In addition, the NMMO in the coagulation bath of the cellulose products may contain a wider variety of impurities, and the concentration of the impurities may be higher. For example, bacteriostatic agents are usually added in cellulose membranes (such as gluconic acid, thymol, chitosan, etc.), and the purification methods required for producing cellulose membranes must be able to completely remove carbohydrates and other impurities soluble in water from the NMMO of the coagulation bath. If the carbohydrates and other soluble impurities cannot be removed, the casting solution enters the coagulation bath after membrane-forming, and these impurities soluble in dilute NMMO aqueous solution on the surface of the membranes including oligosaccharide will redissolve in the NMMO aqueous solution of the coagulation bath, which leads to defects on the surface of the coagulated and precipitated cellulose membranes and even leads to breakage of the membranes in severe cases. The purification method of ion-exchange resins currently used in lyocell fiber production cannot effectively remove various impurities in the coagulation bath including carbohydrate impurities, , as a result, the purification method cannot be used in the preparation of cellulose products that requiring the purification and recovery of high-purity NMMO in the coagulation bath; in addition, some of the coagulation bath solutions of the cellulose products may contain a very high content of inorganic salt impurities. For instance, the concentration of inorganic salts of sodium sulfate and calcium chloride (as pore-forming agents) in the coagulation bath solution of cellulose sponge products and coagulation bath solution of cellulose shower loofah is very high, and even 4-5 times higher than the concentration of NMMO. Such a high concentration of inorganic salts cannot be removed by the method of ion-exchange resin at all.

To economically and stably prepare cellulose products on an industrial scale, it is necessary to find a suitable purification and recovery method that can remove impurities from the NMMO solution of the coagulation bath.

### SUMMARY

A main object of the invention is to provide a purification method and system of N-methylmorpholine N-oxide (NMMO), and a N-methylmorpholine N-oxide (NMMO) obtained thereof to overcome defects in the purification and recovery method of NMMO from the coagulation bath of different cellulose products in the prior art, such as high cost, less environmentally friendly, low purity of the recovered NMMO which may be not reuseable for preparation of cellulose products.

To achieve the above objects, the invention provides a purification method of NMMO, which is used to purify NMMO in a coagulation bath of a cellulose product, the purification method comprises steps of: concentrating the coagulation bath of the cellulose product containing the NMMO until the mass concentration of the NMMO is 56.5% ~ 84.5%, and then performing cooling crystallization between -20°C and 78°C to obtain NMMO hydrate crystals; wherein the mass of the NMMO in the coagulation bath of the cellulose product is 1-50%.

The purification method of NMMO in the invention, wherein the coagulation bath of the cellulose product is: a mixture remaining after the formation of the cellulose product from a cellulose in a NMMO solution; the cellulose product includes at least one of the lyocell staple, lyocell filament, cellulose membrane, cellulose non-woven fabric, cellulose film, cellulose material for cigarette filter, and cellulose sponge.

The purification method of NMMO in the invention, wherein comprising another step before concentrating the coagulation bath of the cellulose product: performing a membrane separation and purification to the coagulation bath of the cellulose product, and the membrane separation and purification includes at least one of the microfiltration, ultrafiltration, nanofiltration and reverse osmosis process.

The purification method of NMMO in the invention, wherein the mass concentration of the NMMO in the coagulation bath of the cellulose product is 56.5% ~ 72% by the concentration, and the temperature of the cooling crystallization is between -20°C ~ 39°C; during the cooling crystallization process, a cooling rate is 1 ~ 4°C/hour; and/or, NMMO hydrate seed crystals are added during the cooling crystallization process to increase the purity of the obtained crystals, and the amount of the NMMO hydrate seed crystals added is above 0.1% of the weight of the NMMO in the coagulation bath of the cellulose product.

The purification method of NMMO in the invention, wherein the cooling crystallization includes at least a primary cooling crystallization. The NMMO hydrate crystals and a crystal mother solution are obtained by the primary cooling crystallization, and the crystal mother solution can continue with the cooling crystallization.

The purification method of NMMO in the invention, wherein after performing or not performing a flocculation and sedimentation of the coagulation bath of the cellulose product, performing the microfiltration and ultrafiltration; a filter aperture of the microfiltration is 0.1 ~ 100µm, and performing the ultrafiltration to a microfiltration filtrate obtained by the microfiltration to obtain an ultrafiltration filtrate, in which the ultrafiltration has molecular weight cut off 1100 ~ 100000 Dalton.

The purification method of NMMO in the invention, wherein performing the nanofiltration to the ultrafiltration filtrate to obtain a nanofiltration filtrate, in which the nanofiltration has molecular weight cut off 150 ~ 1000 Dalton and the NMMO exists in the nanofiltration filtrate.

The purification method of NMMO in the invention, wherein performing a high-pressure reverse osmosis to the nanofiltration filtrate for a pre-concentration, and an operating pressure of the high-pressure reverse osmosis is 50 ~ 83Bar.

To achieve the above objects, the invention further provides a NMMO hydrate crystal obtained by the purification method.

To achieve the above objects, the invention further provides a purification system of NMMO, which is used to purify NMMO in a coagulation bath of a cellulose product, comprising:
at least one of a microfiltration device, an ultrafiltration device and a nanofiltration device, and the coagulation bath of the cellulose product flows into at least one of the microfiltration device, ultrafiltration device and nanofiltration device for processing to obtain a NMMO filtrate;
a crystallization device connected to at least one of the microfiltration device, ultrafiltration device and nanofiltration device, and the NMMO filtrate flows into the crystallization device for a crystallization process;
a control device connected to the crystallization device to control crystallization conditions in the crystallization device.

The purification system of NMMO in the invention, wherein further comprises a high-pressure reverse osmosis device connected to the crystallization device and at least one of the microfiltration device, ultrafiltration device and nanofiltration device, and the NMMO filtrate flows into the high-pressure reverse osmosis device for concentration first and then flows into the crystallization device for crystallization process.

The invention has the following beneficial effects:
The invention provides a method of purifying and recovering NMMO from the coagulation bath of all cellulose products. The method adopts the crystallization process or the process further combined with membrane processes. The purity of the recovered NMMO is high and meets requirements of NMMO purity in the production of various cellulose products. The NMMO recovery rate is high and there is almost no loss. The method is suitable for industrial production.

Furthermore, the purification process of the invention is simple, almost no waste gas, waste water, and solid waste are generated during the recovery and purification process and the purification and recovery cost is low. The invention is an efficient, simple, low-cost and green purification and recovery method for NMMO.

### BRIEF DESCRIPTION OF THE DRAWINGS

Fig. 1 is a comparison diagram of HPLC chromatograms of a lyocell coagulation bath solution before and after the purification using ion-exchange resin by a lyocell manufacturer;
   Wherein, 1 represents the chromatogram of the lyocell coagulation bath solution after the purification using ion-exchange resin, 2 represents the chromatogram of the lyocell coagulation bath solution before the purification using ion-exchange resin;
Fig. 2 is a partial enlarged diagram of Fig. 1;
Fig. 3 is a comparison diagram of HPLC analysis chromatograms of a mixed coagulation bath solution before and after the purification using ion-exchange resin on day 7 of continuous production in the embodiment 1 of the invention;
   Wherein, 1 represents the chromatogram of the mixed coagulation bath solution after the purification using ion-exchange resin, 2 represents the chromatogram of the mixed coagulation bath solution without the purification;
Fig. 4 is a partial enlarged diagram of Fig. 3;
Fig. 5 is a comparison diagram of HPLC analysis chromatograms of a mixed coagulation bath solution without purification and a NMMO after purification by crystallization on day 7 of continuous production in the embodiment 2 of the invention;
   Wherein, 1 represents the chromatogram of the NMMO after purification by crystallization, 2 represents the chromatogram of the mixed coagulation bath solution without purification;
Fig. 6 is a partial enlarged diagram of Fig. 5;
Fig. 7 is a comparison diagram of HPLC analysis chromatograms of a NMMO after membrane process and purification by crystallization on day 7 of continuous production in the embodiment 4 of the invention, and the mixed coagulation bath solution after the purification using ion-exchange resin on day 7 of continuous production in the embodiment 1 of the invention ;
   Wherein, 1 represents the HPLC analysis chromatogram of the NMMO after membrane process and purification by crystallization in embodiment 4, 2 represents the HPLC analysis chromatogram of mixed coagulation bath solution after the purification using ion-exchange resin in embodiment 1;
Fig. 8 is a partial enlarged diagram of Fig. 7.

### DETAILED DESCRIPTION

Embodiments of the invention are described in detail as follows. The embodiments are implemented under the premise of technical solutions of the invention and detailed implementation methods and processes are provided, but the protection scope of the invention is not limited to the embodiments. Experimental methods for which no specific conditions are specified in the embodiments are generally based on conventional conditions. Unless otherwise specified, percentage '%' refers to percentage of mass hereinafter.

The invention provides a purification method of NMMO, which is used to purify NMMO in a coagulation bath of a cellulose product. The purification method comprises steps of: concentrating the coagulation bath of the cellulose product, and then performing cooling crystallization between -20°C and 78°C to obtain NMMO hydrate crystals;

Wherein, the mass concentration of the NMMO in the coagulation bath of the cellulose product is 1-80%, further to be 1~50%, further to be 1-40%, and further to be 1-25%.

Various cellulose products prepared from NMMO solution includes two parts of dissolution of cellulose in a NMMO solution and purification of NMMO solvent in the coagulation bath of a formed cellulose product. The coagulation bath of the cellulose product is: during the preparation of the cellulose product, cellulose is first dissolved in a NMMO aqueous solution with a high concentration (above 85wt%). The prepared cellulose NMMO solution enters the NMMO aqueous solution with a low concentration in which the cellulose product is formed. The cellulose will coagulate and precipitate in the dilute NMMO aqueous solution of the coagulation bath to form corresponding cellulose products, and the remaining dilute NMMO aqueous solution is the coagulation bath of the cellulose product. In a word, the coagulation bath of the cellulose product is the mixture remaining after the cellulose products are formed in the dilute NMMO aqueous solution. Since there exist several oxidative decomposition reactions of cellulose in lyocell process, the coagulation bath of the cellulose product contains a certain amount of impurities. Therefore, the invention provides a method of purifying and recovering NMMO from the coagulation bath of various cellulose products. The method uses cooling crystallization to enable the NMMO and water to form specific hydrate crystals such that achieves the purpose of separation of NMMO from the impurities.

In the invention, cellulose products are not limited to lyocell staple, lyocell filament, cellulose membrane, cellulose non-woven fabric, cellulose film, cellulose material for cigarette filter, cellulose sponge etc., wherein cellulose membrane includes various lamination film such as cellophane, cellulose self-adhesive tape, cellulose preservative membrane, cellulose packaging membrane, cellulose film, cellulose plastic bag, cellulose string, cellulose woven bag, and cellulose separation membrane etc. In addition, the cellulose products in the invention does not limit the method of formation as long as the cellulose products are prepared from NMMO solution. For example, the preparation of the cellulose membrane may use the method of casting and scrapping, the method of extrusion and elongation, the method of extrusion and biaxial elongation (blowing) and etc., to form the membrane.

The NMMO hydrate solids purified and recovered from the coagulation bath of the cellulose product by the crystallization of the invention have high purity and the NMMO recovery rate is high. The obtained NMMO hydrate solids fully meet requirements for production of cellulose products and the purification method of the invention achieves the purpose of purifying and recovering the NMMO from the coagulation baths of cellulose products. The purified and recovered NMMO hydrate solids can be prepared to an aqueous solution with a required concentration, and then reused in the production of the cellulose products.

The coagulation bath of the cellulose product can be directly purified by the method of crystallization to recover the NMMO. However, coagulation baths of different cellulose products contain different variety of impurities and generally contain carbohydrates, which increase the viscosity of the crystallization solution and may also affect the crystallization process and the separation of the NMMO hydrate crystals, which makes it difficult to obtain the NMMO hydrate crystals in high purity and high yields. In addition, these impurities, especially the carbohydrates, will all remain in the crystal mother solution, resulting in a high viscosity of the crystal mother solution, making it difficult to flow and unable to perform crystallization to the crystal mother solution again. And the recrystallization of the crystal mother solution can help further recover the NMMO. Therefore, as a preferred embodiment, if the content of carbohydrate impurities, such as oligosaccharide, etc., in the coagulation bath is too high before purifying the NMMO in the cellulose coagulation bath by the crystallization, membrane separation technology can be used to remove most of the macromolecular impurities such as carbohydrate impurities in advance.

In one embodiment, the invention first removes the carbohydrates in the coagulation bath of the cellulose product and removes or partially removes impurity molecules with relatively large molecular mass in the cellulose coagulation bath at the same time, and then performs cooling crystallization. In another embodiment, the invention adopts membrane separation processes (at least one of the microfiltration, ultrafiltration, nanofiltration and reverse osmosis process) to remove cellulose, hemicellulose, oligosaccharides, disaccharides, polysaccharides with more than two saccharides, some monosaccharides, etc., in the cellulose coagulation bath. Meanwhile, the method of membrane separation processes can also remove the flocculant PAM, some small molecules with relatively large molecular mass such as condensation products of PG and formaldehyde, divalent and higher inorganic salts and other impurities that may be contained in the coagulation bath of the cellulose product. The coagulation bath of the cellulose product pre-treated with the membrane separation processes is concentrated by heating evaporation until the mass concentration is 56.5% ~ 84.5%, and then is performed with the cooling crystallization between -20 °C and 78 °C. In this way, the effects of the carbohydrates and other impurities in the cellulose coagulation bath on the crystallization can be avoided, and the purity and yield of the obtained NMMO hydrate crystals can be further improved.

In one embodiment, performing the microfiltration to the coagulation bath of the cellulose product first to filtrate the solids and obtain a microfiltration filtrate; then performing the ultrafiltration to the microfiltration filtrate to obtain an ultrafiltration trapped concentrate and an ultrafiltration filtrate. In another embodiment, the filter aperture of the microfiltration is 0.1 ~ 100µm and the ultrafiltration has molecular weight cut off 1100 ~ 100000. In this method, the microfiltration can remove the solids in the mixture and the ultrafiltration can remove macromolecular soluble substance in the mixture, and the step-by-step process can improve the efficiency of removing impurities and reduce the loading of the ultrafiltration.

The content of NMMO in the ultrafiltration trapped concentrate and the ultrafiltration filtrate of the invention is essentially the same, for example 150 ~ 250g/l. To improve the recovery efficiency of the NMMO, in one embodiment, the ultrafiltration trapped concentrate is recycled back into the coagulation bath of the cellulose product and further treated with microfiltration etc. again.

In one embodiment, performing the nanofiltration to the ultrafiltration filtrate to obtain a nanofiltration trapped concentrate and a nanofiltration filtrate, while the NMMO presents in the nanofiltration filtrate.

In the above embodiment, the content of NMMO in the nanofiltration trapped concentrate and the nanofiltration filtrate is essentially the same, for example 150 ~ 250g/l.

To improve the recovery efficiency of the NMMO and reduce the waste of the NMMO in the process, the nanofiltration also includes a diafiltration of the nanofiltration trapped concentrate by using water to obtain a nanofiltration diafiltrate, wherein the number of the diafiltration is at least one time, for example can be 2 times, 4 times, 6 times, 8 times, etc. The number of the diafiltration is mainly determined by the content of the NMMO in the nanofiltration trapped concentrate after the diafiltration. When the content of the NMMO in the nanofiltration trapped concentrate after the diafiltration decreases to an acceptable range, for instance the content of the NMMO in the nanofiltration trapped concentrate after the diafiltration is 0 - 10g/l, the diafiltration stops and the nanofiltration trapped concentrate is discharged outside. At meantime, mixing all the obtained nanofiltration diafiltrate together and the content of the NMMO inside is normally 10 ~ 100g/l.

In one embodiment, various impurities with low molecular mass including PG, PG oxides remains in the nanofiltration filtrate or the nanofiltration diafiltrate obtained by the nanofiltration, and results of HPLC analysis shows that the nanofiltration filtrate or the nanofiltration diafiltrate still contains a certain amount of impurities such as PG and PG oxidation products together with other multiple unknown impurities. The nanofiltration filtrate needs further purification for better use in the cellulose production. Hence, the nanofiltration filtrate in the invention needs a further crystallization process.

The invention doesn't specifically limit that the coagulation bath of the cellulose product must undergo all steps of the microfiltration, the ultrafiltration, the nanofiltration and the reverse osmosis process before the crystallization is performed. For instance, performing the microfiltration to the coagulation bath of the cellulose product first and then performing the cooling crystallization to the microfiltration filtrate; performing the microfiltration and ultrafiltration to the coagulation bath of the cellulose product first and then performing the cooling crystallization to the ultrafiltration filtrate; performing the microfiltration, the ultrafiltration and nanofiltration to the coagulation bath of the cellulose product first and then performing the cooling crystallization to the nanofiltration filtrate; performing the microfiltration, the ultrafiltration, the nanofiltration and diafiltration to the coagulation bath of the cellulose product first and then performing the cooling crystallization to the mixture of the nanofiltration filtrate and nanofiltration diafiltrate.

In one embodiment, performing a reverse osmosis process or a reverse osmosis process combined with concentration process to the liquid to be crystallized (such as the coagulation bath of the cellulose product, the microfiltration filtrate, the ultrafiltration filtrate, the nanofiltration filtrate or the mixture of the nanofiltration filtrate and nanofiltration diafiltrate) before the cooling crystallization is performed in the invention, such that the concentration of the NMMO in the abovementioned solutions to be crystallized is increased to above 23% (75-80atm pressure) by the reverse osmosis process, while the concentration of the NMMO in the liquid to be crystallized is 56.5% ~ 84.5% by the evaporation concentration and the crystallization efficiency is improved thereby. In another embodiment, the method of concentration is evaporation, such as vacuum evaporation, reduced pressure evaporation, atmospheric evaporation, etc.

In one embodiment, the invention performs the concentration to the solution to be crystallized and when the mass content of the NMMO in the obtained concentrated solution is 56.5% ~ 72.2%, performing cooling crystallization to the concentrated solution at temperatures between -20 ~ 39°C, so that the obtained NMMO hydrate crystals are 2NMMO·5H₂O (i.e. NMMO·2.5H₂O); when the mass content of the NMMO in the obtained concentrated solution is 72.2% ~ 84.5% (excluding 72.2%), performing cooling crystallization to the concentrated solution at temperatures between 39 ~ 78°C (excluding 39°C), so that the obtained crystalline NMMO is NMMO·H₂O. In other words, the different concentrations of the NMMO in the concentrated solution determine the different crystallization temperatures, and further resulting in different NMMO hydrate crystals.

In one embodiment, consider the cost of industrial purification and operation convenience, the solution to be crystallized is concentrated to 66.5% ~ 72% mass concentration of NMMO, and the cooling crystallization can be performed to the concentrated solution at near-room-temperatures range between 25 ~ 40°C or 25 ~ 36°C (the final crystallization temperature is 25°C and above) in the invention. Under the optimal crystallization conditions, 2NMMO·5H₂O crystal with high purity and a crystallization yield of more than 70% can be obtained after first crystallization; the higher the concentration of the NMMO in the solution before the crystallization and the lower the final crystallization temperature, the higher the obtained NMMO crystallization yield.

Performing the crystallization under the optimal crystallization conditions. the crystallization conditions, such as crystallization yields, cooling rates, and additions of seed crystals, will affect the degree of supersaturation of the metastable state during the crystallization and therefore will affect the purity of the NMMO in crystals.

In one embodiment, a small amount of NMMO seed crystals can be added during crystallization process and the amount of the seed crystals added should be above 0.01%, which is preferably between 0.1% ~ 0.3%, of the weight of the NMMO in the solution to be crystallized. In cooling crystallization, adding seed crystals to induce crystallization can effectively reduce the degree of supersaturation of NMMO in solution, and resulting in crystals with higher NMMO purity.

In one embodiment, the seed crystals added in the invention are small particles of broken crystals with high purity, which are crushed and have a particle size as uniform as possible, and the size of the particles for example ranging from 0.01 to 0.1mm. Seed crystals with relatively large particles or insufficient addition of the seed crystals may lead to a decrease in the purity of crystalline NMMO, and the obtained crystalline NMMO have an uneven distribution of particle size; excessive addition of the seed crystals may lead to relatively small particles of the obtained crystalline NMMO. In addition, the seed crystals should not be added too early, otherwise, the temperature of the solution may be too high, resulting in the possibility to dissolve the crystals due to the concentration of NMMO hydrates in the solution lower than the solubility of NMMO hydrates and the significance of adding the seed crystals will be lost; on the other hand, if the seed crystals are added too late, the temperature of the solution may be too low and the degree of supersaturation of the solution may be too high, which will reduce the actual effect of adding the seed crystals. The timing of adding the seed crystals is generally determined by the NMMO content in the solution to be crystallized and the initial cooling temperature. In one embodiment, seed crystals are added when the solution is cooled to 30-37 °C.

In one embodiment, the seed crystals of the invention are soaked in a high-purity NMMO saturated aqueous solution for at least 1 hour before being added to the crystallization system, which can improve crystallization effect and ensure crystals precipitated in the crystallization system have a uniform size.

In one embodiment, during the cooling crystallization process of the invention, the cooling rate is controlled at 1-4°C/hour; in another embodiment, during the cooling crystallization process of the invention, from initial appearance of crystals in the crystallization system to the crystallization temperature of 30°C, the cooling rate is controlled at 1-2 °C/hour, and after the temperature of the crystallization system drops below 30 °C (for example, at -20-30°C), the cooling rate can be controlled at 3-4°C/hour. In this way, crystal purity can be guaranteed. This is because the cooling rate directly determines the crystallization rate in the cooling crystallization. When the crystallization system is in a relatively high temperature range, the solubility curve of the NMMO hydrates varies greatly. Therefore, in the initial stage of crystallization after the crystals begin to appear, the cooling rate of the solution system should not be too fast, and the growth rate of the crystals should be limited, otherwise it will lead to a high degree of supersaturation of the solution and affect crystal purity.

In one embodiment, after the cooling crystallization system reaches the crystallization termination temperature, crystals and the crystal mother solution are obtained by rapid vacuum filtration of the crystallization solution. The crystals are rinsed with a high-purity NMMO aqueous solution with a concentration of 59.5% to obtain the crystalline NMMO, which are weighed and analyzed for content.

Primary crystallization can generate NMMO hydrate crystals with very high purity. Crystals obtained by the primary crystallization are analyzed and detected by liquid chromatography, and the result shows that various organic impurities present in the original cellulose coagulation bath are not detectable in the crystalline NMMO. The crystals obtained by the primary crystallization are mixed with water to prepare a NMMO aqueous solution with a mass content of 23% and the electrical conductivity of the NMMO aqueous solution may decrease to less than 10.2µs/cm, which means the crystallization can effectively remove inorganic salts impurities in the coagulation bath. Analysis of the total sugar content in the crystal mother solution shows that the content of carbohydrates is low, which indicates that most of the carbohydrate impurities in the coagulation bath of the cellulose product can be removed after the membrane separation process.

The membrane separation process of the invention can remove macromolecular impurities in the coagulation bath of the cellulose product, such as oligosaccharides, etc., which affect the viscosity of the coagulation bath. Consequently, the viscosity of the crystal mother solution is greatly reduced so that the crystal mother solution can achieve multiple concentrations and crystallizations and the crystallization recovery rate of the NMMO can be increased. In an embodiment of the invention, evaporating the mother solution produced by the primary crystallization through vacuum heating until the mass concentration of the NMMO is 69 ~ 72%, and then performing the cooling crystallization. The purity of crystals from the secondary crystallization is lower than the primary crystallization and the crystals from the secondary crystallization can be combined with the coagulation bath as raw materials for the primary crystallization. The combined NMMO recovery rate of the primary crystallization and the secondary crystallization can reach 96%. The viscosity of the mother solution of the secondary crystallization is still not high, and the mother solution can continue to be concentrated and crystallized to further improve the total recovery rate of the NMMO. Finally, a small amount of crystal mother solution containing a relatively high concentration of impurity from the multiple crystallizations can be directly incinerated through an organic incineration method (TO), and waste gas, waste water, and solid waste such as the wastewater with high concentration of salt and COD content, etc. are not generated.

The invention further provides a purification system of NMMO, which is used to purify NMMO in a coagulation bath of a cellulose product, comprising:
a crystallization device configured to crystallization process of the coagulation bath of the cellulose product;
a control device connected to the crystallization device to control crystallization conditions in the crystallization device.
In one embodiment, the purification system of NMMO in the invention further comprises:
at least one of a microfiltration device, an ultrafiltration device and a nanofiltration device connected to the crystallization device and the control device, and the coagulation bath of the cellulose product flows into at least one of the microfiltration device, ultrafiltration device and nanofiltration device for processing to obtain a filtrate. The filtrate flows into the crystallization device for crystallization process.

In another embodiment, the purification system of NMMO in the invention further comprises:
a microfiltration device, and the coagulation bath of the cellulose product flows into the microfiltration device to filter solids and obtain a microfiltration filtrate; an ultrafiltration device is connected to the microfiltration device, and the microfiltration filtrate flows into the ultrafiltration device for ultrafiltration process and an ultrafiltration trapped concentrate and an ultrafiltration filtrate are obtained;
a nanofiltration device is connected to the ultrafiltration device and the crystallization device respectively, and the ultrafiltration filtrate flows into the nanofiltration device for nanofiltration process and a nanofiltration trapped concentrate and a nanofiltration filtrate are obtained, while the NMMO presents in the nanofiltration filtrate. The nanofiltration filtrate flows into the crystallization device for crystallization process.

In another embodiment, the purification system of NMMO in the invention further comprises a high-pressure reverse osmosis device connected to the crystallization device and at least one of the microfiltration device, the ultrafiltration device and the nanofiltration device. The NMMO filtrate flows into the high-pressure reverse osmosis device for concentration and then flows into the crystallization device for crystallization.

The method in the invention can purify and recover the NMMO solvents in the coagulation bath of all cellulose products, the purity of the recovered NMMO is high (nearly reaches the purity of commercially available NMMO products) and the recovery rate is high. The whole recovery process of the coagulation bath discharges almost no wastewater. In addition, since carbohydrate impurities with good water solubility such as oligosaccharide, etc. produced during the dissolution of the cellulose can be removed in time, the cheap broad-leaved forest pulps containing a high hemicellulose content can be used as raw materials instead of just the expensive coniferous forest pulps and cotton pulps, which greatly reduces the production cost of the cellulose product. Besides, the purity of the cellulose solution prepared by the recovered NMMO is high due to the high purity of the recovered NMMO, and in a result, the quality of the cellulose product is also improved.

The purification and recovery processes of the NMMO in the cellulose coagulation bath produced during the preparation of different cellulose products are described in detail by the detailed embodiment below, including the preparation of the spiral cellulose flat membranes (cellophane) by the method of extrusion and elongation, the preparation of the cellulose tubular membranes by the method of extrusion and biaxial elongation (blowing), and the preparation of the cellulose sponges. Embodiments below shows that the method disclosed by the invention can effectively remove almost all impurities in the NMMO solution from the coagulation bath of the below cellulose products and realizes the purification and recovery of the NMMO well. Furthermore, the purification method disclosed by the invention can also apply to the production of other cellulose products prepared from the NMMO solution (for example, the cellulose flat membranes prepared by the method of scrapping, the cellulose packaging membranes, the cellulose non-woven fabrics, the cellulose cigarette filters, the cellulose films, the cellulose shower loofah, the cellulose sponges, etc.). The preparation of these cellulose products may require addition of some required organic and inorganic functional materials (for instance inorganic salt pore-forming agents, flame retardants, bacteriostatic (anti-bacterial) agents, glittering agents, electrically and thermally conductive substances, etc.). The method in the invention can effectively and timely remove various organic and inorganic impurities in the NMMO of the coagulation bath including carbohydrates, and the purpose of the purification and recovery of the NMMO can be achieved.

The first embodiment relates to the preparation of the spiral cellulose flat membranes (cellophane) by the method of extrusion and elongation.

According to the preparation method of the cellulose solution, preparing a casting solution with a cellulose mass concentration of 9%, a mass concentration of PG is 0.01%, and a mass concentration of bacteriostatic agent of gluconic acid is 0.1%. The cellulose membranes are prepared by an extrusion and elongation membrane manufacturing device, the concentration of the NMMO in its first coagulation bath is 55-56%, and the concentration of the NMMO in the second coagulation bath is 5%. The concentration of the NMMO after mixing the first coagulation bath and the second coagulation bath is around 23% (hereinafter refers to mixed coagulation bath). The solution of the mixed coagulation bath contains various impurities including above impurities and the bacteriostatic agent of gluconic acid. The purification and recovery of the NMMO in the mixed coagulation bath uses the method of anion-exchange resin and cation-exchange resin and the recovered NMMO is reused in the production. On the third day of continuous production, the surface of the membrane begins to appear unevenly concaves and the membrane begins to appear holes on the seventh day, which significantly reduce the membrane forming ratio. Detection of the NMMO solution of the mixed coagulation bath for the seventh day shows that the sugar content is 284ppm and the sugar content in the NMMO solution after the process of ion-exchange resin is still 258ppm, which means the ion-exchange resin cannot effectively remove carbohydrates in the NMMO solution of the coagulation bath. In addition, comparing HPLC analysis chromatograms of the NMMO solution of the mixed coagulation bath before and after the processing using ion-exchange resin, it shows that there are still other multiple impurities unremovable by the ion-exchange resin, inevitably resulting in the continuous accumulation of the impurities in the casting solution, including carbohydrates with low molecules that are easily dissolved in the coagulation bath solution. When the membranes of the cast solution formed by extrusion and elongation enter the coagulation bath, these impurities, including carbohydrates, existing on the surface of the membranes are dissolved in the coagulation bath solution again, which leads to damages of the membranes. In the meantime, other impurities unremovable by the ion-exchange resin, besides the carbohydrate impurities, also accumulate. In addition, other properties of the cellulose membranes may be damaged.

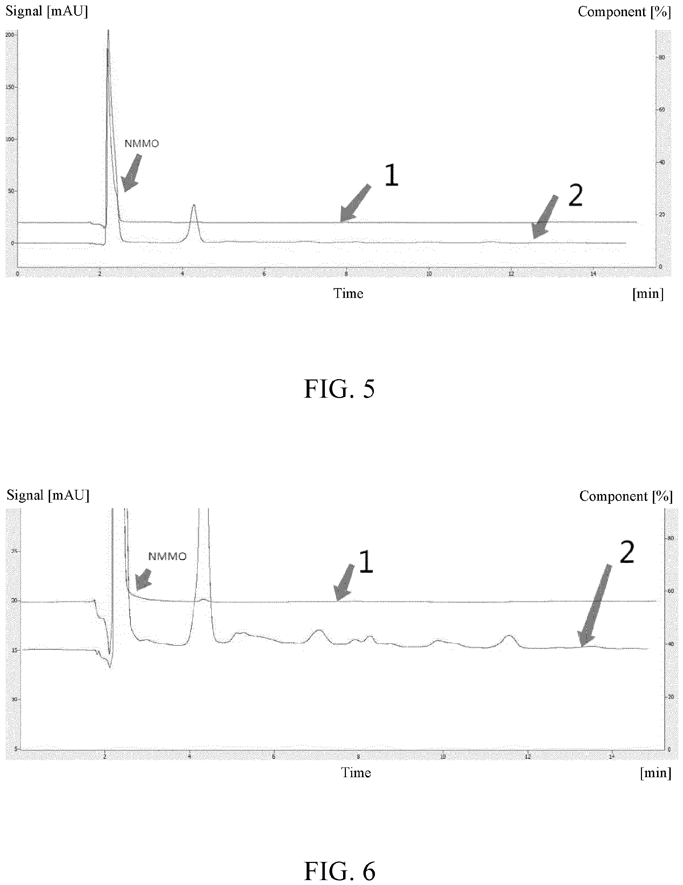

The above NMMO solution of the mixed coagulation bath on the seventh day of continuous production is directly performed with the method of crystallization to purify and recover the NMMO inside. HPLC chromatography analysis shows that the purity of the recovered NMMO is close to commercially available NMMO products and the recovered NMMO contains almost no impurities that are contained in the NMMO solution of the coagulation bath, including carbohydrate impurities (Fig. 5 and 6). However, since these impurities, especially the carbohydrates, basically all remain in the crystal mother solution, resulting in a relatively high viscosity of the crystal mother solution. The crystal mother solution is hard to flow and multiple crystallizations cannot be performed. The total NMMO recovery rate is relatively low.

The spiral cellulose flat membranes can be prepared by the above method but the NMMO solution of the coagulation bath is purified by the method of crystallization. Since the content of the carbohydrate impurities in the coagulation bath solution at the initial production is low and the crystallization can timely remove almost all impurities including the carbohydrate impurities, the content of the carbohydrate impurities in the coagulation bath solution during the whole production period is maintained at a very low level. Therefore, using only the purification method of crystallization can still obtain NMMO with high recovery rate, and the effect of the purification by crystallization is good. On the seventh day of continuous production, the prepared cellulose membranes don't have any concaves and damages. The purification method of crystallization is obviously better than the purification method using ion-exchange resins.

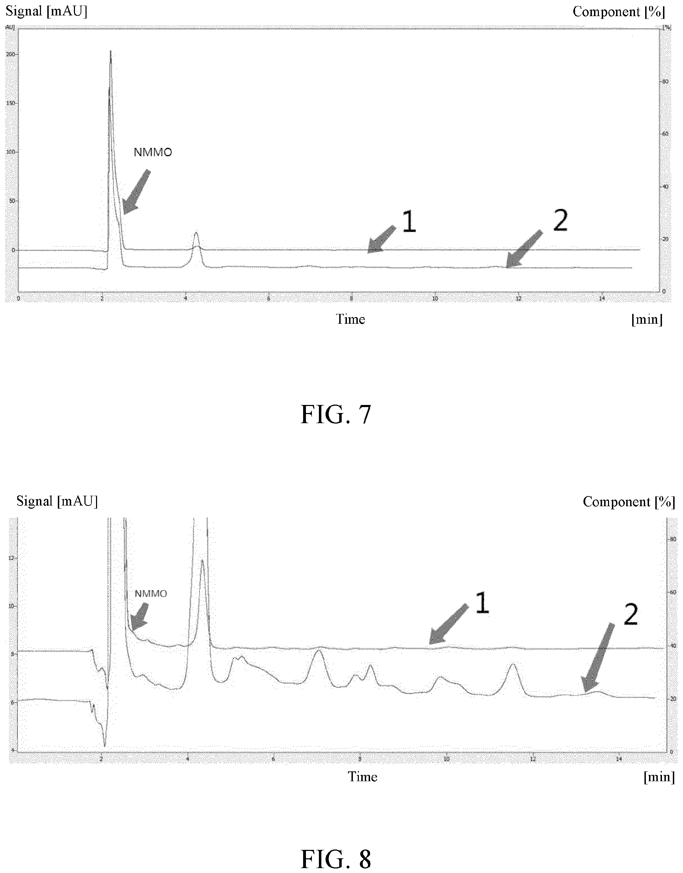

The spiral cellulose flat membranes can be prepared by the above method but the NMMO solution of the mixed coagulation bath is purified by the purification method combining membrane separation processes and crystallization. The membrane separation processes (by microfiltration membrane, ultrafiltration membrane, and nanofiltration membrane) remove carbohydrate impurities such as oligosaccharides, disaccharides, polysaccharides with more than two saccharides, some monosaccharides, etc., condensation products from the reaction of PG and formaldehyde, divalent and higher inorganic salts and other impurities. However, analysis result shows that multiple other unknow impurities and impurities with a low molecular mass, including bacteriostatic agents, monosaccharides, PG and PG oxides, cannot be separated by the membrane completely. Most of the impurities still remains in the coagulation bath solution and the coagulation bath solution is then further concentrated until the mass concentration of NMMO is 70.2% by heating evaporation under reduced pressure. The cooling crystallization is performed between 25 ~ 40°C and 2NMMO·5H₂O crystals with very high purity can be obtained. All impurities that cannot be separated by the separation membrane combinations can be effectively removed by the crystallization method. Furthermore, comparing to the individual ion-exchange resin process and individual crystallization process, the viscosity of the crystal mother solution is greatly reduced, and multiple crystallizations of the mother solution can be performed to recover the NMMO inside. Using the separation membrane combinations and the crystallization technology to purify and recover the NMMO in the mixed coagulation bath for a continuous production of 168 hours (7 days), and the impurity content in the mixed coagulation bath is essentially the same as at the beginning. The purity of the purified and recovered NMMO is still close to commercially available NMMO products. The obtained NMMO crystals are dissolved with water to form an aqueous solution with NMMO concentration of 23% and the electrical conductivity of the NMMO aqueous solution is 2.8µs/cm. Comparing with the cellulose membranes produced on the first day, the properties of the membrane produced on the seventh day including mechanical properties such as the tensile strength, breaking elongation, etc., moisture permeability of the membrane, etc. basically remains the same, which indicates the concentration of the impurities in the coagulation bath solution is maintained at a very low level after 7 days production, accumulation of the impurity concentration is not found, and the production and quality of the cellulose membranes are not affected. The NMMO solution of the mixed coagulation bath on the first day without the purification process is analyzed and the result shows the sugar content is 9ppm, while the sugar content of the NMMO solution of the mixed coagulation bath on the seventh day is still 9ppm, which means carbohydrates can be effectively and timely removed by the purification of the coagulation bath. The HPLC analysis of the mixed coagulation bath solutions of the seventh day with the purification only by ion-exchange resin and with the purification by the method combining membrane preparation processes and crystallization (Fig. 7 and 8) shows that the impurity content in the coagulation bath solution purified by the method combining separation membrane combinations and the crystallization is significantly lower than that of the above purification method using ion-exchange resin. Also, the total sugar content is significantly lower than that of the above purification method using ion-exchange resin (9ppm vs. 258ppm).

The second embodiment relates to the preparation of the cellulose tubular membranes by the method of extrusion and biaxial elongation (blowing).

The cellulose tubular membranes are produced by an extrusion and biaxial elongation (blowing) device and the process conditions for blowing are as follows: the casting solution is a NMMO solution with a cellulose mass concentration of 9% (which is prepared by the preparation method of cellulose solution), and the NMMO in the coagulation bath solution is purified and recovered by the purification method combining the separation membrane combinations and the crystallization. Devices with ultrafiltration membrane and the nanofiltration membrane in separation membrane combinations (including ultrafiltration membrane and nanofiltration membrane) remove impurities dissolved in the NMMO of the coagulation bath, such as carbohydrates with two or more saccharides, condensation products from the reaction of PG and formaldehyde, divalent inorganic salts, etc. A device with reverse osmosis membrane can concentrate a coagulation bath solution with a NMMO mass concentration of 5% to the NMMO mass concentration of 25.7%. The NMMO mass concentration is then concentrated to 69.8% by heating under reduced pressure and the cooling crystallization is performed between 25 ~ 40°C. The NMMO purified and recovered from the coagulation bath has a very high purity. The NMMO is colorless and transparent, containing almost no impurity. The viscosity of the crystal mother solution is very low and the NMMO in the crystal mother solution can be recovered to the maximum extent by multiple crystallizations of the crystal mother solution. The blowing device operates continuously for 168 hours (7 days), mechanical properties such as the longitudinal tensile strength, breaking elongation, etc., and moisture permeability (water flux) of the cellulose membranes produced on the first day and the seventh day basically remain the same, which means there is not accumulation of impurities in the coagulation bath and the preparation of the cellulose tubular membranes is not affected.

The third embodiment relates the preparation of cellulose sponges.

During the preparation of the cellulose sponges, a pore-forming agent of anhydrous Na₂SO₄ solid that is 3 times the weight of the cellulose NMMO solution is usually added. Hence, the NMMO aqueous solution of the coagulation bath contains a very high concentration of Na₂SO₄. In this embodiment, the coagulation bath of the cellulose sponge is a NMMO aqueous solution with a mass concentration of 2%, and the mass concentration of around 8.5% of Na₂SO₄ in the coagulation bath. The device with nanofiltration membrane in the separation membrane combinations (by microfiltration membrane, ultrafiltration membrane, nanofiltration membrane, reverse osmosis membrane) can trap and remove the Na₂SO₄. The Na₂SO₄ in the nanofiltration trapped concentrate can be purified by the conventional purification method of mirabilite using crystallization and sodium sulfate decahydrates can be obtained. The sodium sulfate decahydrates are dehydrated by heating to obtain anhydrous sodium sulfate solids (to be reused). The nanofiltration filtrate is a NMMO aqueous solution with a NMMO mass concentration of around 2%. The NMMO aqueous solution can be concentrated until the NMMO mass concentration is 23.1% by the device with reverse osmosis membrane and can be further concentrated until the mass concentration is 69.9% by heating evaporation under reduced pressure. The NMMO aqueous solution is purified by crystallization between 25 ~ 40°C. HPLC chromatography analysis shows that the purity of the obtained 2NMMO·5H₂O crystals is very high. At the same time, the crystals are added with water to prepare a NMMO aqueous solution with a mass concentration of 23% and the electrical conductivity is 8.2µs/cm.

The result of the embodiment shows that the purification method disclosed by the invention can still purify and recover the NMMO solvents in the coagulation bath of the cellulose sponge products containing a high concentration of sodium sulfate inorganic salts.

In one detailed embodiment, the purification method combining the membrane combinations and the crystallization comprises steps of:
Pre-treatment of the cellulose coagulation bath by the separation membrane combinations. The separation membrane combinations include microfiltration, ultrafiltration, nanofiltration and reverse osmosis.

The cellulose coagulation bath can be performed or not be performed with flocculation and sedimentation, and the use of a microfiltration system can remove mechanical impurities before entering into the ultrafiltration system to remove macromolecular impurities and colloids. The ultrafiltration membrane has molecular weight cut off 5000 ~ 100000; the NMMO mass concentration in the ultrafiltration trapped concentrate after the diafiltration is lower than 1% and the ultrafiltration trapped concentrate is discharged as waste liquid. The ultrafiltration filtrate enters a nanofiltration unit to trap molecular weight of 150 ~ 1000, by which the soluble impurities such as oligosaccharide and metal complexes can be removed and some of the monosaccharides can be removed as well. The method of diafiltration is used to improve the yield of the NMMO. The NMMO mass concentration in the nanofiltration trapped concentrate after the diafiltration is lower than 1% and the nanofiltration trapped concentrate is discharged as waste liquid. The NMMO mass concentration in the nanofiltration filtrate after nanofiltration is lower than 15%. The NMMO mass concentration in the nanofiltration filtrate is concentrated to higher than 20% by the reverse osmosis membrane, and the concentrated solution then enters a thermal evaporation unit; the evaporated liquid containing no NMMO is treated as clean water and flows back to the nanofiltration and ultrafiltration to be used a diafiltrate or water supplement for the coagulation bath.

The coagulation bath solution pre-treated by the separation membrane combinations with a NMMO mass concentration above 20% is further concentrated until the NMMO mass concentration is 66.5 ~ 72% by heating evaporation under reduced pressure.

The purification by crystallization is performed under the optimal crystallization conditions. The coagulation bath solution of the cellulose products with a NMMO mass concentration of 66.5 ~ 72% at around 40°C is cooled down to a temperature at which seed crystals will not dissolve after being added (the temperature is known as the temperature of the initial appearance of crystals), and then a small amount of the seed crystals is added. The seed crystals are preferably broken crystals of 2NMMO·5H₂O in the form of small particle, which are pre-soaked in a high-purity NMMO saturated solution. The broken crystals in the form of small particle are soaked in the NMMO saturated solution for at least 1 hour, and the amount of seed crystals added is 0 ~ 1.0% of the weight of the NMMO in the coagulation bath, which is preferably 0.1-0.3%. After adding seed crystals, continuing to cool down the coagulation bath slowly. The cooling rate is controlled at 1-2°C/ hour from the temperature of the initial appearance of crystals to 30°C. When the temperature drops below 30 °C, the cooling rate can be appropriately accelerated to 3-4 °C/hour, and the crystallization termination temperature is 25 °C. Crystals in the form of large particle with an even distribution of particle size and low impurity content can be obtained under this crystallization conditions. After rapid filtration of the crystal solution at a high temperature, the mother solution after the filtration is collected and weighed. The crystals are rinsed with a high-purity NMMO aqueous solution with a mass concentration of 59% for 3 times and the crystals are collected to be weighed and analyzed for crystal purity. An aqueous solution with NMMO mass concentration of 23% is prepared by adding water to the crystals, and the total sugar content, total impurity content, the electrical conductivity, content of copper and iron metal ions, etc. are detected.

The crystal mother solution is concentrated by heating evaporation under reduced pressure until the NMMO mass concentration is about 70% and the crystal mother solution is performed with the crystallization again. The obtained crystal mother solution can be performed with multiple concentrations and crystallizations to recover the NMMO to the maximum extent. Generally, 3 times crystallizations can recover above 99% of the NMMO in the cellulose coagulation bath.

Crystallization separation may adopt the method of batch crystallization or continuous crystallization. For the method of continuous crystallization, the crystallization operation can be carried out with different forms of continuous crystallizers, such as OSLO continuous crystallizer, DTB continuous crystallizer, etc. Since seed crystals are not require in the method of continuous crystallization, a very high primary crystallinity (above 95%) can be obtained easily, and the operation is simple and convenient, the method of continuous crystallization is quite suitable for the purification and recovery of the NMMO in the cellulose coagulation bath in the large-scale industrialized production.

The cellulose pulp used in the embodiment of the invention: broad-leaved wood pulp (dissolving pulp) produced in China, degree of polymerization (DP) = 584 (cuprammonium), and mass fraction of the α- cellulose is 90.5%.

The preparation method of the cellulose solution in the embodiment is as follows: pulverizing the wood pulp, weighing and collecting a required weight of pulverized wood pulp and 2NMMO·5H₂O crystals (the NMMO mass concentration is 72.2%). After adding the pulverized wood pulp and 2NMMO·5H₂O crystals in a 10-liter reactor, heating the solution to 75-80°C and placing the solution at a constant temperature for 1 hour (the pulp has been fully swelled). Then propyl gallate (PG) and other specific auxiliaries (such as the bacteriostatic agent of gluconic acid), which are equivalent to 0.1% of the weight of the solution, are added. The solution is heated to 95-100°C and is stirred at a vacuum degree of 0. 1MPa. The solution is evaporated and concentrated to a cellulose/NMMO/H₂O solution that is brown, transparent and viscous. Letting the prepared cellulose solution stand for 1 hour at the vacuum degree of 0. 1MPa for deaeration, and the cellulose solution can be used for the preparation of membranes is obtained.

The method of preparing cellulose flat membranes by extrusion and elongation in the embodiment is: the casting solution in the embodiment of the invention is a NMMO solution with a cellulose mass concentration of 9%. The screw pump pumps the casting solution to a self-made spinneret die of a film pulling device, and the extrusion flow rate of the casting solution is 80ml/minute. The spinneret die is provided with a long nozzle. The nozzle length is 200mm and the nozzle gap is adjustable within a range of 0.1 ~ 0.4mm. In the embodiment, the gap is 0.2mm. The casting solution is extruded into membrane by the nozzle and the extruded membrane enters a first coagulation bath after passing through an air gap of 100mm. The NMMO mass concentration of the first coagulation bath is 55 ~ 65% and the temperature of the first coagulation bath is 30°C. The membrane in the first coagulation bath is laterally pulled and stretched to a membrane width of 300mm by clamps of pulling chains on both side of the membranes. The membrane is pulled forward by rollers at a pulling rate of 2.5m/minute and then enters a second coagulation bath. The NMMO mass concentration of the second coagulation bath is 5% and the temperature of the second coagulation bath is controlled at 20°C. Then cellulose membrane pulled by the rollers enters a water bath, a plasticizer bath (glycerin aqueous solution) and a drying chamber in order. Variations of the retention time in each part can be realized by adjusting the running distance of the membrane in each part. The membrane stays in the first coagulation bath for 3 minutes, stays in the second coagulation bath for 3 minutes, stays in the water bath for 5 minutes, and stays in the plasticizer bath for 10 minutes. The mass concentration of glycerin in the plasticizer bath is 18 ~ 20% and the temperature of the water bath and the plasticizer bath are both controlled at 25°C. The drying chamber of the cellulose flat membrane is divided into three temperatures for drying, for example 20 minutes at 50°C, 20 minutes at 70°C and 20 minutes at 90°C. Variations of the retention time in each part can be realized by adjusting the running distance of the membrane in each part. The NMMO mass concentration in the mixed coagulation bath obtained by mixing the first coagulation bath and the second coagulation bath is around 23%, and the mixed coagulation bath can be recycled after purification, recovery and concentration.

The method of preparing cellulose tubular membranes by extrusion and biaxial elongation (blowing) in the embodiment is: the casting solution in the embodiment of the invention is a NMMO solution with a cellulose mass concentration of 9%. The screw pump pumps the casting solution to a self-made spinneret die of a blowing device. The spinneret die is provided with a spinneret ring with a diameter of 200mm. The gap of the spinneret ring is adjustable within a range of 0.2 ~ 1.5mm. In the embodiment of the invention, the gap is 0.5mm. An air vent and two liquid draft tubes are provided at the center of the spinneret ring. Compressed air flows into the lumen of the tubular membranes through the air vent and the blowup ratio can be adjusted by air pressure. One of the draft tubes is used for continuously introducing the coagulation bath (which is a deionized water in the embodiment) while the other draft tube is used for continuously draining the coagulation bath out from the lumen of the tubular membranes (which can be sucked out by negative pressure). The extrusion flow rate of the casting solution is 30ml/minute. The casting solution is extruded from the gap of the spinneret ring to form the closed tubular membrane of the casting solution and the air gap between the spinneret ring and the surface of the coagulation bath is 100mm. The NMMO mass concentration of the coagulation bath outside the membranes is controlled at 5%, the NMMO mass concentration of the coagulation bath inside the tubular membranes is also controlled at 5%, and the temperature of the coagulation bath is controlled at 20°C. The membranes are pulled forward by rollers at a pulling rate of 1.8m/minute and the blowup rate is controlled at 1.25 (adjusting the air pressure inside the membranes by controlling the air compression). Cellulose membrane pulled by the rollers enters the coagulation bath, a water bath, a plasticizer bath (glycerin aqueous solution) and a drying chamber in order. Variations of the retention time in each part can be realized by adjusting the running distance of the membrane in each part. The membrane stays in the coagulation bath for 3 minutes, stays in the water bath for 5 minutes, and stays in the plasticizer bath for 10 minutes. The mass concentration of glycerin in the plasticizer bath is 18 ~ 20% and the temperatures of the coagulation bath, water bath and the plasticizer bath are all controlled at 25°C. The drying chamber of the tubular membranes is divided into three temperatures for drying, for example drying for 20 minutes at 50°C, drying for 20 minutes at 70°C and drying for 20 minutes at 90°C. The coagulation bath solutions inside and outside the membranes are mixed together for purification and the purified NMMO is recycled.

The preparation method of cellulose sponges in the embodiment: preparing a cellulose NMMO solution with a concentration of 7% according to the above preparation method of the cellulose solution. Then adding shredded degrease cotton with a degree of polymerization above 1000 at 80°C as sponge strength enhancer, and the amount added is 50% of the weight of the wood pulp. Stirring or kneading for 1 hour to mix well and then adding colorless sodium sulfate solid particles which is 3 times the weight of the cellulose solution. Oversized particles (greater than 40 mesh) and undersized particles (smaller than 200 mesh) of the sodium sulfate are filtered out before adding and the sodium sulfate is pre-heated to 90°C. The sodium sulfate works as a pore-forming agent. Sodium sulfate and the cellulose solution is mixed well together by kneader. The mixture is poured to a 30mm x 50mm x 100mm rectangular mold and then leave for 1 hour until the temperature cool down to room temperature. Then the moulded mixture of the sodium sulfate and the cellulose solution are soaked in the coagulation bath for 2 hours and the NMMO mass concentration in the coagulation bath is 2.2%. The coagulation bath solution is squeezed out of the cellulose sponges formed in the coagulation bath. Then, the sponge is put into a water bath to rinse the residual NMMO and inorganic salts out of the sponge. After water is drained out of the sponge, the sponge is further dried under reduced pressure for 24 hours at 80°C. The NMMO in the coagulation bath are purified and recovered for recycling.

The crystallization method in the embodiment: the coagulation bath solution of cellulose products with a NMMO mass concentration between 68.5-72% at a temperature of about 40°C is cooled down to a temperature at which the seed crystals will not dissolve after being added (the temperature is known as the temperature of the initial appearance of crystals), and then a small amount of seed crystals is added. The seed crystals are preferably broken crystals of 2NMMO·5H₂O in the form of small particle, which are pre-soaked in a high-purity NMMO saturated solution. The broken crystals in the form of small particle are soaked in the NMMO saturated solution for at least 1 hour, and the amount of seed crystals added is 0-1.0% of the weight of the NMMO in the coagulation bath, which is preferably 0.1-0.3%. After adding seed crystals, continuing to cool down the coagulation bath solution slowly. The cooling rate is controlled at 1 - 2°C/ hour from the temperature of the initial appearance of crystals to 30°C. When the temperature drops below 30 °C, the cooling rate can be appropriately accelerated to 3-4 °C/hour, and the crystallization termination temperature is 25 °C. Crystals in the form of large particle with an even distribution of particle size and low impurity content can be obtained under this crystallization conditions. After rapid vacuum filtration of the crystal solution while it is hot, the mother solution after the filtration is collected and weighed. The crystals are rinsed with a high-purity NMMO aqueous solution with a mass concentration of 59% for 3 times and the crystals are collected to be weighed and analyzed for crystal purity.

The detection of iron ion content and copper ion content in the embodiment: the instrument is Thermo Fisher ICP-MS.

The detection of total sugar content in the embodiment: using the analytical method for total sugar disclosed in the 'Study on Lyocell fiber from a cheap pulp with high hemicellulose content', which is a PhD dissertation of the Donghua University written by Huiru Zhang.

The analytical method of organic impurities in the coagulation bath solution in the embodiment: using liquid chromatography (HPLC) analysis, and chromatographic column is: AichromBond-1, C18, 5µm 4.6x150mm; mobile phase: 0.2% phosphate buffer, pH = 2.9; flow rate of mobile phase: 1 ml/min; column temperature: 35 °C; UV detection wavelength: 200nm and 220nm, the detections are performed separately; sample size: 20µl; detection time: 30 minutes.

The testing method of membrane performance in the embodiment:
Testing instrument: Fiber Tensile Tester, Shanghai New Fiber Instrument Co., Ltd.

Testing of membrane strength: the dried membrane is cut into 90mm x 5mm sample strips in both longitudinal and transverse directions and is tested in accordance with national standard GB/T14337-2008 of testing method for the tensile properties of the sample strips by using the Fiber Tensile Tester. The tensile speed is 100mm/min and the clamping distance of the test is 10mm. Each sample is tested for 5 times and the average value is taken.

Thickness of membrane: 10 points are taken randomly on the surface of the membrane sample by plastic film thickness gauge for thickness measurement and the average value is taken.

The testing method of moisture permeability (water flux) of the cellulose membranes: test is performed in accordance with national standard GB1037-88 (Test method for water vapor transmission of plastic film and sheet - Cup method).

All samples analyzed in embodiments of the invention are analyzed and tested as described above.

### Embodiment 1

The embodiment mainly studies the effect of purification and recovery of NMMO in the coagulation bath of the cellulose membrane by using ion-exchange resins.

The preparation of the cellulose membrane uses the preparation method of the spiral flat membranes by extrusion and elongation. The mass concentration of the cellulose solution is 9%, the mass concentration of PG is 0.1%, the mass concentration of bacteriostatic agent of thymol is 0.1%, the NMMO mass concentration of the first coagulation bath is 55 ~ 65%, and the NMMO mass concentration of the second coagulation bath is 5%. The first and second coagulation bath solution are mixed together for purification and recovery and the mass concentration of the coagulation bath after mixing is around 23% (hereinafter refers to mixed coagulation bath). The method for purification and recovery of the NMMO in the mixed coagulation bath is the method of ion-exchange resin currently used in the production of lyocell fiber. The NMMO solution of the coagulation bath pass through the anion-exchange resin column and cation-exchange resin column successively. Anion-exchange resin columns contain macroporous strong base resins while the cation-exchange resin columns contain macroporous weak acid resins. When the electrical conductivity of the coagulation bath solution after passing through the anion-exchange resin columns and cation-exchange resin columns is ≥ 20µs/cm, ion-exchange resins can be determined exhausted. The regeneration method of the exhausted ion-exchange resins is: the regeneration of cation-exchange resins requires a hydrochloric acid aqueous solution with mass concentration of 4-6% that is 6 times the volume of the resins for rinsing the resin column, while the regeneration of anion-exchange resins requires a NaOH aqueous solution with mass concentration of 4-6% that is 6 times the volume of the resins. After rinsing with acid solution or alkali solution, the resin column is then rinsed by high purity water until the water from rinsing the resin column has a pH value of 6-8, which means the rinsing and the regeneration is complete. The regenerated ion-exchange resins can be used to process coagulation bath solution again. The purified and recovered dilute NMMO solution is concentrated to an aqueous solution with NMMO mass concentration around 72% by a conventional depressurization and evaporation device, and the aqueous solution returns to be used in preparing the cellulose solution (according to the above preparation method of the cellulose solution).

Purifying and recovering the NMMO in the mixed coagulation bath using ion-exchange resin and preparing the spiral cellulose flat membranes by extrusion and elongation. Continuously preparing the cellulose membrane for 7 days. Concaves and uneven thickness are observed on the surface of the prepared flat membranes on the 3^{rd} day. The longer the continuous production time, the more obvious the concaves and uneven thickness are. On the 7^{th} day, the membranes start to break and tear constantly, and the membrane forming ratio decreases significantly. Samples of the mixed coagulation bath on the 1^{st} day, the 3^{rd} day, the 5^{th} day and the 7^{th} day are collected for total sugar content analysis separately, and the NMMO solution of the mixed coagulation bath after purification by ion-exchange resin on the 7^{th} day is collected for total sugar content analysis. Results are shown in Table 1.

**Table 1. Total sugar content in the NMMO solution of the mixed coagulation bath with days of continuous production**

| Days of continuous production | The 1^{st} day | The 3^{rd} day | The 5^{th} day | The 7^{th} day | The 7^{th} day (after purification by ion-exchange resin) |
|---|---|---|---|---|---|
| Total sugar content in the mixed coagulation bath solution (ppm) | 37 | 110 | 192 | 284 | 258 |

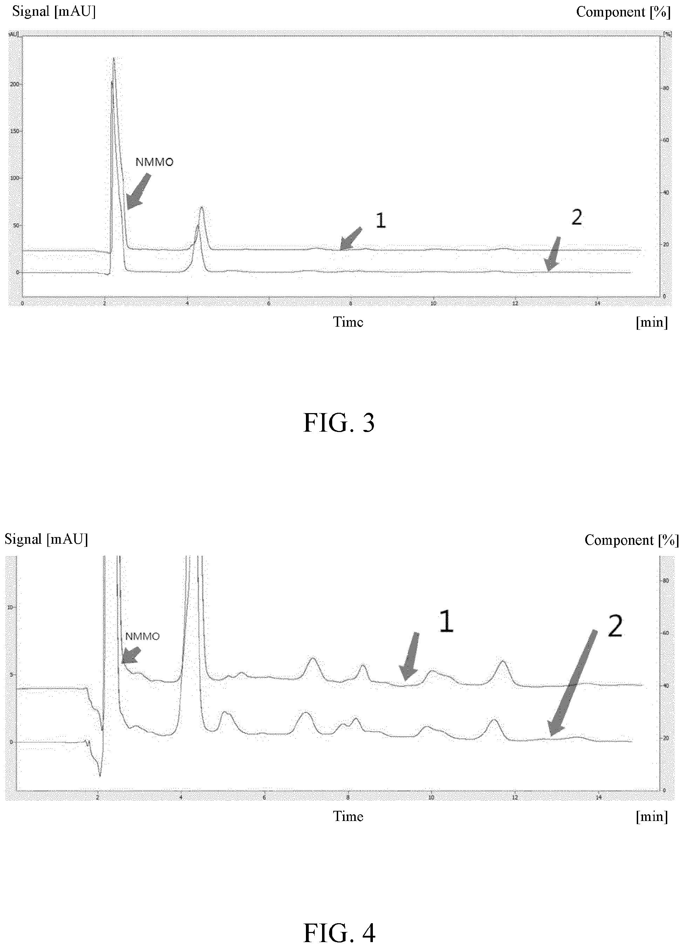

As shown in table 1, the total sugar content in the NMMO solution of the mixed coagulation bath on the 7^{th} day after purification by ion-exchange resin is slightly lower than the total sugar content before the purification. However, most of the carbohydrates are still not removed, resulting in continuous accumulation of carbohydrates from the decomposition of cellulose and hemicellulose in membrane production system. When the cellulose dissolves at high temperatures to form a casting solution, a small amount of cellulose and hemicellulose may decompose and break into various organic impurities, including soluble oligosaccharides, if these impurities cannot be timely removed during the purification, it will inevitably lead to the continuous accumulation of organic impurities, such as carbohydrates, in the coagulation bath. Comparison of HPLC analysis chromatograms of the mixed coagulation bath on the 7^{th} day before and after the purification using ion-exchange resin shows that a lot of organic impurities in the coagulation bath cannot be removed by the ion-exchange resins (refers to Fig. 3 and 4). Table 1 also shows that as the days of continuous production increase, the total sugar content in the coagulation bath solution continues to increase. When the cellulose membranes enter the coagulation bath solution, organic impurities, including the carbohydrates in the membrane tissues that are soluble in the coagulation bath solution will be re-dissolved from the membranes, inevitably resulting in defects on the membrane surface and even membrane breakage in severe cases.

### Embodiment 2

The embodiment mainly studies the effect of direct purification of the coagulation bath by crystallization after the concentration by conventional heating evaporation under reduced pressure without pre-treatment of the separation membrane combinations.

The mixed coagulation bath solution on the 7^{th} day in the embodiment 1 (the NMMO mass concentration is around 23%) has an electrical conductivity of 82µs/cm and a total sugar content of 284ppm. The mixed coagulation bath solution is concentrated until the NMMO mass concentration is 70.1% by the conventional heating evaporation under reduced pressure for crystallization and a crystallization yield of 82% is obtained. The obtained NMMO crystals dissolve in high-purity water and are diluted to a solution with the NMMO mass concentration of 23%. The electrical conductivity of the solution is 2.8µs/cm and the total sugar content in the solution is lower than 1.0ppm. The solution is analyzed and detected by HPLC chromatography and the organic impurities is undetectable (refers to Fig. 5 and 6).

Residual crystal mother solution after the crystallization has a relatively high viscosity. The crystal mother solution is concentrated until the NMMO mass concentration is 68.0% by the heating evaporation under reduced pressure again for crystallization and a crystallization yield of 64% is obtained. However, the viscosity of the second crystal mother solution is very high, which makes the solution difficult to flow and unable to be performed with the concentration and crystallization again. Detection of the second crystal mother solution shows that the total sugar content is 5.2%. The obtained NMMO crystals dissolve in high-purity water and are diluted to a solution with the NMMO mass concentration of 23%. The electrical conductivity of the solution is 3.4µs/cm and the total sugar content in the solution is 3.5ppm. The solution is analyzed and detected by HPLC chromatography, and the results show that the purity of the crystalline aqueous solution is very high, and the organic impurities is almost undetectable.

Therefore, using the crystallization method to purify and recover the NMMO solvent in the mixed coagulation bath of the process for preparing flat membranes by using extrusion and elongation can effectively remove organic impurities, including carbohydrates, in the mixed coagulation bath, and can remove inorganic salt impurities in the mixed coagulation bath at the same time. The electrical conductivity after the purification decreases greatly. In the embodiment, the mixed coagulation bath solution has a total crystallization yield of 93.5% after the second crystallization. Since the crystal mother solution contains a high concentration of carbohydrates, it is difficult to further improve the NMMO crystallization yield by crystallization, which leads to a loss of a small amount of NMMO.

### Embodiment 3

The embodiment mainly studies the effect of purification and recovery of the NMMO in the coagulation bath by using purification method of crystallization alone when the content of sugar impurities in the coagulation bath solution is relatively low.

Preparing the cellulose flat membranes according to the method in embodiment 1 but using the purification method of crystallization to purify and recover the NMMO solvent in the mixed coagulation bath. The content of the carbohydrate impurities produced by the cellulose decomposition at initial production stage is generally low. Purifying the NMMO solvent in the coagulation bath by using the crystallization alone at this stage, and the carbohydrate impurities has relatively small effects on the recovery of the NMMO crystals. The embodiment adopts the same process conditions as the extrusion and elongation method in embodiment 1 to prepare the spiral cellulose flat membranes but using the purification method of crystallization alone to purify and recover the NMMO solvent in the mixed coagulation bath. The production continues for 7 days and samples of the coagulation bath solution on the 1^{st}, 3^{rd}, 5^{th}, and 7^{th} day are collected for total sugar content analysis separately (refers to Table 2). Taking the crystallization purification of the mixed coagulation bath solution produced on the 7^{th} day as an example, the mixed coagulation bath solution with a NMMO mass concentration of 23% is concentrated until the NMMO mass concentration is 70.1% by heating evaporation under reduced pressure for crystallization and a crystallization yield of 83% is obtained. The first crystal mother solution is concentrated until the NMMO mass concentration is 70% by heating evaporation under reduced pressure again for the second crystallization and a crystallization yield of 80% is obtained. The viscosity of the second crystal mother solution is still not high. The second crystal mother solution is concentrated until the NMMO mass concentration is 69.8% by heating evaporation under reduced pressure again for the crystallization and a crystallization yield of 77% is obtained. The total crystallization yield of three crystallizations is 99.2% (sum of the three crystallizations).

**Table 2. Total sugar content in the NMMO solution of the mixed coagulation bath with days of continuous production (purification method of crystallization)**

| Days of continuous production | The 1^{st} day | The 3^{rd} day | The 5^{th} day | The 7^{th} day |
|---|---|---|---|---|
| Total sugar content in the mixed coagulation bath solution (ppm) | 8 | 9 | 8 | 8 |

As shown in Table 2, for preparing the cellulose flat membranes by extrusion and elongation, when broad-leaved forest pulps are used as raw material of the cellulose membrane and the purification method of crystallization alone to purify and recover the NMMO solvent in the mixed coagulation bath is adopted, the sugar content in the coagulation bath solution is maintained at a very low and stable level during the 7 days of the continuous production. The produced cellulose membranes have uniform surfaces and a stable product quality. At the initial production stage of the cellulose membrane in the embodiment, the content of carbohydrate impurities in the coagulation bath solution is low. Purifying and recovering the NMMO solvent in the mixed coagulation bath solution by using the purification method of crystallization alone and the total NMMO crystallization yield can still reach 99.2%, which means using the purification method of crystallization alone can meet the need of purification and recovery of the NMMO in the coagulation bath solution with a low concentration of carbohydrate impurities.

### Embodiment 4

The embodiment mainly studies the effect of purification and recovery of the NMMO solvent in the coagulation bath of the cellulose membrane by combining the separation membrane combinations and the crystallization technology.

The embodiment adopts the same process as the extrusion and elongation method in embodiment 1 to prepare the spiral flat membranes but the purification and recovery of the NMMO in the mixed coagulation bath is performed by a method combining the separation membrane combinations and the crystallization technology. The mixed coagulation bath solution is pre-treated by the separation membrane combinations to remove the carbohydrate impurities inside and is then performed with crystallization method to further purify and recover the NMMO solvent in the mixed coagulation bath. The separation membrane combinations consist of microfiltration, ultrafiltration and nanofiltration. The microfiltration is used to remove dust and mechanical particles, while the main purpose of the ultrafiltration and nanofiltration is to remove carbohydrate impurities which have a relatively large effect on the concentration of the solution. The mixed coagulation bath solution after the pre-treatment of the separation membranes is concentrated by heating evaporation under reduced pressure and is then purified by the crystallization to obtain 2NMMO·5H₂O crystals. The casting solution in the embodiment is prepared by the preparation method of cellulose solution above and the NMMO crystals obtained by the purification method of crystallization are directly returned for the preparation of cellulose solutions. The mass concentration of the cellulose solution of the casting solution in the embodiment is 9%, the mass concentration of PG is 0.1%, the mass concentration of thymol is 0.1%, and the NMMO mass concentration of the mixed coagulation bath solution is about 23%.

For the continuous production of the cellulose membranes in the embodiment, the uneven thickness and breakage on the surface of the membranes are not found as the increase of the continuous membrane production time. The production of the membranes continues for 7 days and the quality and breakage ratio of the membrane are obviously better than the membranes in embodiment 1. Samples of the mixed coagulation bath on the 1^{st}, 3^{rd}, 5^{th}, and 7^{th} day are collected separately for the analysis of the total sugar content and the electrical conductivity of the sample solutions (refers to Table 3).

The mixed coagulation bath on the 7^{th} day after the pre-treatment of the separation membrane combinations is further concentrated until the NMMO mass concentration is 70.1% by heating evaporation under reduced pressure for crystallization and a crystallization yield of 83% is obtained. The first crystal mother solution is concentrated until the NMMO mass concentration is 69.9% by heating evaporation under reduced pressure again for crystallization and a crystallization yield of 80% is obtained. The viscosity of the second crystal mother solution is not high. The second crystal mother solution is concentrated until the NMMO mass concentration is 69.0% by heating evaporation under reduced pressure again for crystallization and a crystallization yield of 73% is obtained. The total NMMO crystallization yield of three crystallizations is 99.1%.

Membrane products produced on the 1^{st}, 3^{rd}, 5^{th}, and 7^{th} day are collected separately, and the longitudinal strength, breaking elongation and moisture permeability of the produced cellulose membranes are tested according to the aforementioned method respectively.

**Table 3. Total sugar content in the mixed coagulation bath solution and properties of the produced cellulose membranes with days of continuous production (the purification method combining the separation membrane combinations and the crystallization)**

| | The 1^{st} day | The 3^{rd} day | The 5^{th} day | The 7^{th} day |
|---|---|---|---|---|
| Total sugar content (ppm) | 9 | 8 | 8 | 9 |
| Electrical conductivity (µs/cm) | 71 | 77 | 73 | 73 |
| Membrane longitudinal strength (MPa) | 74 | 72 | 76 | 76 |
| Membrane breaking elongation (%) | 20 | 22 | 20 | 20 |
| Water flux (ml/cm²h) | 2.1 | 2.2 | 2.0 | 2.1 |

As shown in table 3, the purification method combining the separation membrane combinations and the crystallization can effectively remove various impurities, including carbohydrate impurities, in the mixed coagulation bath solution, and the content of the various impurities in the mixed coagulation bath solution is significantly lower than that in the mixed coagulation bath solution of embodiment 1 using the purification method of ion-exchange resins. The production continues for 7 days and quality indicators, such as mechanical properties and moisture permeability of the membrane, are basically stable. In the membrane production, the quality and the breakage ratio of the membrane are obviously better than that in embodiment 1 using the purification method of ion-exchange resins. Comparing HPLC analysis chromatograms of the mixed coagulation bath of the cellulose membrane on the 7^{th} day in embodiment 1 and the mixed coagulation bath of the cellulose membrane on the 7^{th} day in the embodiment 4, the purification method combining the separation membrane combinations and the crystallization is obviously better than the purification method of ion-exchange resins in embodiment 1. The content of the organic impurities in the coagulation bath is significantly decreased. (refers to Fig. 7 and 8).

### Embodiment 5

The embodiment mainly studies the effect of purification and recovery of the NMMO solvent in the coagulation bath of the cellulose tubular membranes, which are prepared by the method of extrusion and biaxial elongation (blowing), by the purification method combining the separation membrane combinations and the crystallization.

The NMMO solvent in the coagulation bath solution of the cellulose tubular membranes, which are prepared by the method of extrusion and biaxial elongation (blowing), in the embodiment is purified and recovered by using the same method that combining the separation membrane combinations and the crystallization as in embodiment 5. Cellulose tubular membranes are prepared in accordance with the process conditions of the abovementioned extrusion and biaxial elongation (blowing). The production continues for 7 days, and significant concaves and unevenness on the surface of the cellulose membrane are not found. Various properties of the produced membranes are stable, and membrane breakages are not found. For the coagulation bath solution pre-treated by the separation membrane combinations in the embodiment, the separation membrane combinations comprise another reverse osmosis membrane besides the microfiltration, ultrafiltration and nanofiltration. The reverse osmosis process can concentrate the NMMO mass concentration of the coagulation bath solution from 5% to 25.7%, which greatly reduces energy consumption of the following concentrations.

Samples of the coagulation bath solutions and the membrane products produced on the 1^{st}, 3^{rd}, 5^{th}, and 7^{th} day are collected separately, and the total sugar content and electrical conductivity of the coagulation bath solutions as well as the longitudinal strength, breaking elongation and moisture permeability (water flux) of the membranes are tested respectively.

**Table 4. Total sugar content in the coagulation bath solution and properties of the produced cellulose membranes with days of continuous production (the purification method combining the separation membrane combinations and the crystallization)**

| | The 1^{st} day | The 3^{rd} day | The 5^{th} day | The 7^{th} day |
|---|---|---|---|---|
| Total sugar content (ppm) | 7 | 12 | 11 | 12 |
| Electrical conductivity (µs/cm) | 64 | 68 | 66 | 62 |
| Membrane longitudinal strength (MPa) | 89 | 92 | 93 | 91 |
| Membrane transverse strength (MPa) | 58 | 56 | 55 | 57 |
| Membrane breaking elongation (%) | 19 | 20 | 18 | 19 |
| Water flux (ml/cm²h) | 1.6 | 1.5 | 1.4 | 1.5 |

As shown in table 4, the purification method of the coagulation bath solution combining the membrane combinations and the crystallization can effectively and timely remove various impurities, including carbohydrates, in the NMMO solution of the coagulation bath. Mechanical properties and water flux (moisture permeability) of membranes produced for 7 consecutive days are stable.

### Embodiment 6

The embodiment mainly studies the effect of purification and recovery of the NMMO solvent in the coagulation bath solution of the cellulose sponge products by the purification method combining the separation membrane combinations and the crystallization.

The cellulose sponge products in the embodiment are prepared in accordance with the abovementioned preparation method for cellulose sponges. During the preparation of the cellulose sponges, anhydrous sodium sulfate as a pore-forming agent that is added at 3 times the weight of the cellulose solution needs to be added. The amount of the inorganic salt added is very high, as a result, a large amount of water has to be added to dilute the coagulation bath. Therefore, the content of sodium sulfate in the coagulation bath solution is very high, while the NMMO concentration of the coagulation bath is very low. The NMMO concentration of the coagulation bath solution in the embodiment is 2.2% while the concentration of sodium sulfate is 8.5%. Such high concentration of inorganic salts cannot be removed by ion-exchange resin at all.

The coagulation bath solution in the embodiment is first pre-treated by the separation membrane combinations. The nanofiltration membrane can trap divalent and higher inorganic salts while the NMMO can pass through the nanofiltration membrane, and the sodium sulfate and the NMMO can be effectively separated thereby. The NMMO solution passing through the nanofiltration membrane is further concentrated by the reverse osmosis membrane. For the coagulation bath solution pre-treated by the separation membrane combinations in the embodiment, the NMMO mass concentration can reach 23.1% and the electrical conductivity is 1841µs/cm. It shows that the separation membrane combinations in the invention not only can effectively remove carbohydrate impurities in the coagulation bath, but also can effectively remove the high concentration of inorganic salts with high valence in the coagulation bath. In addition, the coagulation bath solution with a low NMMO concentration can be concentrated until the NMMO mass concentration is above 23% in a cheap way. The nanofiltration trapped concentrate is a highly concentrated sodium sulfate solution, which can be purified by the conventional purification method of mirabilite using crystallization to recover the sodium sulfate.

The coagulation bath pre-treated by the separation membrane combinations is concentrated until the NMMO mass concentration is 70.1% by conventional heating evaporation under reduced pressure. Purifying and recovering the NMMO in the coagulation bath by crystallization and a crystallization yield of 81.5% is obtained. The obtained NMMO crystals are added with high-purity water and are diluted to an aqueous solution with a NMMO mass concentration of 23%. The electrical conductivity of the solution is 8.2µs/cm. The crystal mother solution is concentrated until the mass concentration is 69.8% for crystallization again and a crystallization yield of 78.6% is obtained. A total crystallization yield of 96% can be obtained by the sum of the first crystallization and the second crystallization. Crystals obtained by the second crystallization are added with water and are diluted to an aqueous solution with a NMMO mass concentration of 23%. The electrical conductivity of the solution is 10.2µs/cm. The viscosity of the second crystal mother solution is still not high. The second crystal mother solution can be further concentrated for crystallization to improve the crystallization yield of the NMMO. Crystals obtained from two crystallizations can be reused to prepare the cellulose solutions.

### Industrial application

In summary, the invention provides a purification and recovery method of the NMMO in the coagulation bath solution of the cellulose products. Depending on the composition of the coagulation bath solution of the cellulose products, the purification method of combining the separation membrane pre-treatment and crystallization or of crystallization alone can be used. For the purification method of combining the separation membrane pre-treatment and the crystallization, the coagulation bath solution of the cellulose is first pre-treated by the separation membrane combinations to remove the carbohydrate impurities and divalent and higher inorganic salts, which affect the viscosity of the solution, in the coagulation bath. Then almost all the organic and inorganic impurities in the NMMO solution of the coagulation bath are removed by the crystallization to obtain high-purity NMMO hydrate crystals. The crystals are added with water to prepare a NMMO aqueous solution with a mass concentration of 23%. The electrical conductivity of the NMMO aqueous solution is 2.8 ~ 10.2µs/cm, and the NMMO mass content in the crystals is 72.2%. The crystals can be directly returned for the preparation of the cellulose solution using the swelling method. The crystal mother solution of the NMMO can be crystallized multiple times and a NMMO recovery rate of above 99% can be achieved. A small amount of residual crystal mother solution containing a large amount of impurities can be incinerated through an organic waste liquid incineration method (TO). No waste gas, waste water, and solid waste are generated during the entire process. The method provided in the invention can be used for the purification and recovery of the NMMO in the coagulation bath of all cellulose products prepared from the NMMO solvent. Since the ion-exchange resins are not used, environmental problems caused by the wastewater produced by the regeneration of ion-exchange resins and spent ion-exchange resins are completely solved. In addition, if the NMMO concentration of the coagulation bath is low, the reverse osmosis membrane can be added in the separation membrane combinations. The NMMO solution of the coagulation bath with a low concentration can be concentrated until the mass concentration is above 23% by the reverse osmosis membrane at a low cost, which greatly reduces energy consumption of the following concentrations. The purification method in the invention has advantages of low production cost, simple industrialized operation and wide applicability.

Of course, the invention can still have various other embodiments, and without departing from the spirit and essence of the invention, the person skilled in the art can make various corresponding modifications and variations according to the invention, but these corresponding modifications and variations shall belong to the protection scope of the claims of the invention.

## Claims

1. A purification method of NMMO, wherein the purification method is used to purify NMMO in a coagulation bath of a cellulose product, the purification method comprises steps of:
concentrating the coagulation bath of the cellulose product containing the NMMO until the mass concentration of the NMMO is 56.5% ~ 84.5%, and then performing cooling crystallization between -20°C and 78°C to obtain NMMO hydrate crystals; wherein the mass concentration of the NMMO in the coagulation bath of the cellulose product is 1-50%.

2. The purification method of NMMO according to claim 1, wherein the coagulation bath of the cellulose product is: a mixture remaining after the formation of the cellulose product from a cellulose in a NMMO solution; the cellulose product includes at least one of the lyocell staple, lyocell filament, cellulose membrane, cellulose non-woven fabric, cellulose film, cellulose material for cigarette filter, and cellulose sponge.

3. The purification method of NMMO according to claim 1, wherein comprising another step before concentrating the coagulation bath of the cellulose product: performing a membrane separation and purification to the coagulation bath of the cellulose product, and the membrane separation and purification includes at least one of the microfiltration, ultrafiltration, nanofiltration and reverse osmosis process.

4. The purification method of NMMO according to claim 1, wherein the mass concentration of the NMMO in the coagulation bath of the cellulose product is 56.5% - 72% by the concentration, and the temperature of the cooling crystallization is between -20°C - 39°C; during the cooling crystallization process, a cooling rate is 1 - 4°C/hour; and/or, NMMO hydrate seed crystals are added during the cooling crystallization process to increase the purity of the obtained crystals, and the amount of the NMMO hydrate seed crystals added is above 0.1% of the weight of the NMMO in the coagulation bath of the cellulose product.

5. The purification method of NMMO according to claim 1, wherein the cooling crystallization includes at least a primary cooling crystallization and the NMMO hydrate crystals and a crystal mother solution are obtained by the primary cooling crystallization, and the crystal mother solution can continue with the cooling crystallization.

6. The purification method of NMMO according to claim 1, wherein after performing or not performing a flocculation and sedimentation of the coagulation bath of the cellulose product, performing a microfiltration and an ultrafiltration; a filter aperture of the microfiltration is 0.1 ~ 100µm, and performing the ultrafiltration to a microfiltration filtrate obtained by the microfiltration to obtain an ultrafiltration filtrate, in which the ultrafiltration has molecular weight cut off 1100 - 100000 Dalton.

7. The purification method of NMMO according to claim 6, wherein performing a nanofiltration to the ultrafiltration filtrate to obtain a nanofiltration filtrate, in which the nanofiltration has molecular weight cut off 150 ~ 1000 Dalton and the NMMO exists in the nanofiltration filtrate.

8. The purification method of NMMO according to claim 7, wherein performing a high-pressure reverse osmosis to the nanofiltration filtrate for a pre-concentration, and an operating pressure of the high-pressure reverse osmosis is 50 ~ 83Bar.

9. A NMMO hydrate crystal obtained by the purification method according to any one of claims 1-8.

10. A purification system of NMMO, which is used to purify NMMO in a coagulation bath of a cellulose product, wherein comprising:
at least one of a microfiltration device, an ultrafiltration device and a nanofiltration device, and the coagulation bath of the cellulose product flows into at least one of the microfiltration device, ultrafiltration device and nanofiltration device for processing to obtain a NMMO filtrate;
a crystallization device connected to at least one of the microfiltration device, ultrafiltration device and nanofiltration device, and the NMMO filtrate flows into the crystallization device for a crystallization process;
a control device connected to the crystallization device to control crystallization conditions in the crystallization device.

11. The purification system of NMMO according to claim 10, wherein further comprises a high-pressure reverse osmosis device connected to the crystallization device and at least one of the microfiltration device, ultrafiltration device and nanofiltration device, and the NMMO filtrate flows into the high-pressure reverse osmosis device for concentration first and then flows into the crystallization device for crystallization process.
